# EUROPEAN PATENT APPLICATION

(11) **EP 3 928 823 A1**
(43) Date of publication of application: **29.12.2021**
(21) Application number: 20182690.6
(22) Date of filing: 26.06.2020
(51) Int. Cl.: A61M 25/02

(54) **A MEDICAL DEVICE SECUREMENT SYSTEM**

(71) Applicant: Latch Medical, 4 Dublin (IE)
(72) Inventor: BERTOLLO, Nicky, Dublin, 24 (IE); BENSON, Ronan, Dublin, D24 W53F (IE); COSTELLO, Mark, Co. Mayo, F31R623 (IE)
(74) Representative: FRKelly

(57) **Abstract**

The invention provides a medical device securement system (10) for releasably anchoring a medical device such as a catheter hub (H) to the skin (S) of a patient in a manner which prevents or reduces the likelihood of the catheter hub from being displaced or dislodged, the system (10) comprising a main body having a first section (14) and a second section (16) displaceable relative to one another between an undeployed state and a deployed state, a skin adhering element in form of a first and a second array of microneedles projecting from a tissue contacting surface of the first and second sections (14,16), a retention device (24) provided on the main body for receiving and engaging the medical device, wherein the first section (14) comprises a first base defining the tissue contacting surface and a closure member (20) hingedly articulated relative thereto between a closed and opened position.

## Description

### Field of the invention

This invention relates to a medical device securement system, and in particular but not exclusively for releasably anchoring a medical device such as a catheter hub or the like to the skin of a patient in a manner which prevents or significantly reduces the likelihood of the catheter hub and associated catheter from being displaced and/or dislodged under the influence of external forces such as tension applied to the catheter hub via the catheter.

### Background of the invention

In a significant number of medical procedures, and during periods of monitoring or convalescence of patients, it is necessary to securely anchor various forms of medical device to the patient, and at various anatomical sites which may vary in size, shape, surface conditions, etc. One of the most common forms of device to be so anchored is a catheter, which are commonly used to deliver or drain fluids from the patient, in addition to acting as a guide conduit for various other interventional medical devices such as stent delivery systems, etc. Such catheters are frequently connected through a so called catheter hub, to which different or replacement catheters can be quickly and easily connected, with the remaining downstream portion of the catheter remaining in situ in the patient. Such catheter hubs there serves as a logical and practical point of connection by which the catheter may be secured to the skin or other tissue of the patient.

It is generally highly desirable and often critical that the catheter, catheter hub or other medical device is not inadvertently tensioned or displaced, as this may lead to a loss of function and resulting complications for the patient or treatment being administered.

While there are numerous known systems for securing catheters and catheter hubs or the like to a patient, these prior art systems often require the use of straps, Velcro^{®} or adhesive or adhesive pads to be applied to the skin, which can be difficult and time consuming to apply, in particular depending on the location on the body at which the anchor is required to be located. Tensioning straps to adequately secure the device in position can put undue pressure on the surrounding tissue and can be uncomfortable for the patient, potentially chaffing the surrounding tissue, and creating general discomfort. The efficacy of adhesives can vary significantly depending on the condition of the patient's skin, and has a tendency to degrade over time.

It is therefore an object of the present invention to provide a robust yet simple medical device securement system for securing a medical device such as a catheter hub or the like to a tissue substrate and in particular but not exclusively the skin.

### Summary of the invention

According to a first aspect of the invention there is provide a medical device securement system comprising a main body having a first section and a second section displaceable relative to one another to translate the system between an undeployed state and a deployed state; a tissue adhering element provided on at least one of a tissue contacting surface of the first section and a tissue contacting surface of the second section; a retention device provided on the main body for receiving and engaging the medical device; wherein the first section comprises a first base defining the tissue contacting surface and a closure member hingedly articulated relative to the first base between a closed position defining an enclosure between the first base and the closure member in which the retention device is located and in which the medical device may be at least partially contained, and an open position permitting access to the enclosure to facilitate insertion/removal of the medical device.

Preferably, the tissue adhering element comprises a first array of microneedles projecting from the tissue contacting surface of the first section and a second array of microneedles projecting from the tissue contacting surface of the second section.

Preferably, the first and second sections are displaceable relative to one another to translate the system into the deployed state only when the closure member is in the closed position.

Preferably, the first section comprises a first coupling and the second section comprises a second coupling engageable with the first coupling to lock the system in the deployed state.

Preferably, the first and second couplings are arranged to be engaged with one another as the system is displaced into the deployed state.

Preferably, the first and second couplings are arranged for irreversible interlocking with one another.

Preferably, the closure member comprises a primary portion hingedly articulated to the first base and a secondary portion displaceable relative to the primary portion between a retracted position and an extended position, the secondary portion and the second section being engaged when the system is in the deployed state, the secondary portion being displaceable from the retracted position to the extended position while remaining engaged with the second section such as to displace the system into the undeployed state.

Preferably, the closure member comprises a release mechanism operable to retain the primary and secondary portions in the retracted position and to permit the primary and secondary portions to be displaced into the extended position upon actuation of the release mechanism.

Preferably, the release mechanism comprises a detent lock on one of the primary or secondary portion and a corresponding socket on the other of the primary or secondary portion, the detent lock being biased into the socket when the primary and secondary portions are in the retracted position and manually displaceable out of the socket to permit the primary and secondary portions to be displaced into the extended position.

Preferably, the secondary portion is displaceable linearly between the retracted and extended positions in order to effect linear displacement of the second section relative to the first section to translate the system into the undeployed state.

Preferably, the primary and secondary portions are separable when in the extended position.

Preferably, the secondary portion is rotationally displaceable between the retracted and extended positions, the second section and the secondary portion defining a first set of complementary engaging surfaces which translate rotary motion of the secondary portion from the retracted to the extended positions into linear motion of the second section relative to the first section to translate the system into the undeployed state.

Preferably, the first set of complementary engaging surfaces comprise a cam surface on one of the first or second section and a corresponding follower surface on the other of the first or second section.

Preferably, the first section and the second sections comprise a second set of co-operating engaging surfaces whose relative motion during displacement of the system from the undeployed to the deployed state effects articulation of the closure member from a partially closed state to a fully closed state, wherein in the partially closed state one or more abutments on the closure member are located above or out of a plane of the tissue contacting surfaces and in the fully closed state are located in or below the plane of the tissue contacting surfaces.

Preferably, the second set of co-operating engaging surfaces comprise a ramp on one of the first or second section and a corresponding follower on the other of the first or second section, the ramp and follower being positioned such that displacement of the first section relative to the second section displaces the follower along an inclined face of the ramp such as to effect articulation of the closure member from the partially closed state to the fully closed state.

Preferably, the closure member comprises a plurality of stabilising feet defining the abutments.

Preferably, the plurality of stabilising feet are each defined by a lower or free edge of a pair of sidewalls of the closure member.

Preferably, the plurality of feet are disposed outboard of the microneedles.

Preferably, the second section comprises a second base engaged with and capable of displacement relative to the first base.

Preferably, the second base defines a channel into which the first base is engaged, the channel being dimensioned to permit the first base to be displaced longitudinally within the channel.

Preferably, the retention device comprises a pair of posts in spaced relationship to one another.

Preferably, the retention device is arranged to enable the relative position of the pair of posts to be adjusted.

Preferably, the closure member defines a pair of opposed openings through which the medical device may extend.

Preferably, the first and second section are displaceable relative to one another in a first direction and one or more microneedles of the first array overlap with one or more microneedles of the second array in a second direction substantially perpendicular to the first direction when the system is in the deployed state.

Preferably, the securement system according is adapted to facilitate occlusion of the tissue adhering element.

Preferably, the two tissue contacting surfaces are displaceable into opposing engagement with one another such as to occlude the tissue adhering element.

According to a second aspect of the present invention there is provided a method of releasably securing a medical device to tissue with a securement system, the method comprising the steps of engaging the medical device with a retention device on a body of the system; displacing a closure member of the securement system from an open position exposing the retention device to a closed position at least partially occluding the retention device and medical device; displacing a first section of the body relative to a second section of the body to translate the securement system from an undeployed state to a deployed state; and securing to the tissue a tissue adhering element provided on at least one of a tissue contacting surface of the first section and a tissue contacting surface of the second section before, during, or after displacing the securement system into the deployed state.

Preferably, the tissue adhering element comprises a first array of microneedles projecting from the first section and a second array of microneedles projecting from the second section, the method comprising the steps of inserting the first array and the second array of microneedles into the tissue; displacing the first section relative to the second section to translate the securement system from the undeployed state to the deployed state, wherein the first and second array of microneedles effect localised deformation of the tissue surrounding the microneedles when the body is in the deployed state.

Preferably, the method comprises stabilising the securement system against the tissue by displacing one or more stabilising feet on the first section into contact with the tissue and maintaining said contact while the system is in the deployed state.

Preferably, the method comprises the step of utilising the relative motion of the first and second sections during translation of the system into the deployed state to effect the displacement of the one or more stabilising feet into contact with the tissue.

Preferably, the method comprises subsequently releasing the medical device from the tissue, the method comprising displacing a secondary portion of the closure member relative to a primary portion of the closure member from a retracted position to an extended position, the secondary portion and the second section being engaged when the system is in the deployed state, and displacing the secondary portion from the retracted position to the extended position while the secondary portion remains engaged with the second section such as to displace the system into the undeployed state.

Preferably, the method comprises the step of displacing the two tissue contacting surfaces into opposing engagement with one another such as to occlude the tissue adhering element.

As used herein, the term "tissue adhering element" is intended to mean any element or combination of elements that are capable of securing a device to animal tissue, in particular skin, such as one or more layers or patches of adhesive, one or more arrays of microneedles, or any other suitable functional equivalent.

As used herein, the term or "microneedle" is intended to mean a feature or needle/barb which is of a particular dimension, generally in the range of 100-3,000 micrometres (µm) in length or height, and may include for example a "microneedle" which can be used as a barb and/or as a combined barb and drug delivery or bio-sensing system.

As used herein, the relative term "above" is intended to mean above or out of contact with a tissue surface such as the skin, while the relative term "below" is intended to mean in contact with and pressed lightly into said tissue surface such as to be effectively below the normal plane of the tissue surface.

As used herein, the term "closure member" is intended to mean one or more parts or components, or a portion of one or more larger parts or components, which may be displaced from an open position into a closed position in which the closure member covers or surrounds an object in order to capture, hold, immobilise and/or retain that part such as to provide stability to the object and/or resist forces acting on the object.

### Brief description of the drawings

The present invention will now be described with reference to the accompanying drawings, in which:
Fig. 1a illustrates a plan view of a preferred embodiment of a securement system according to the invention, in an un-deployed state and with a closure member in an open position and having a catheter hub secured thereto;
Fig. 1b illustrates a side elevation of the securement system of Fig. 1a which shows the securement system out of contact with a section of skin;
Fig. 2a illustrates a plan view of the securement system with the closure member in a partially closed position;
Fig. 2b illustrates a side elevation of the arrangement illustrated in Fig. 2a which shows the securement system positioned on the section of skin;
Fig. 3a illustrates a further side elevation of the securement system with the closure member in the partially closed position;
Fig. 3b illustrates a front elevation of the arrangement of Fig. 3a.
Fig. 4a illustrates a side elevation with a first section and a second section of the securement system being displaced into further engagement with one another and translating the system towards a deployed state;
Fig. 4b illustrates a front elevation from of Fig. 4a;
Fig 5a illustrates a side elevation with the first section and second sections fully displaced into engagement with one another to translate the system into the deployed state;
Fig 5b illustrates a front elevation of Fig. 5b;
Fig. 6 illustrates a sectioned side elevation of Figs. 3a;
Fig. 7 illustrates a sectioned side elevation of Figs. 4a;
Fig. 8 illustrates a sectioned side elevation of Figs. 5a;
Fig. 9a illustrates a plan view of the securement system with a primary and a secondary portion of the closure member displaced into an extended position to facilitate removal of the securement device from the section of skin;
Fig. 9b illustrates a side elevation of the arrangement illustrated in Fig. 9a which shows arrays of microneedles of the system being withdrawn from the section of skin;
Fig. 10a illustrates a plan view of the securement system with the primary and a secondary portion separated and the closure member rotated back into the open position;
Fig. 10b illustrates a side elevation of the arrangement illustrated in Fig. 10a and which shows the microneedles having being fully withdrawn from the skin and the securement system removed therefrom;
Fig. 10c illustrates a perspective view from beneath of the system, showing the first and second sections fully extended following translation into the undeployed state;
Fig. 10d illustrates an enlarged view of a portion of the arrangement shown in Figure 10c;
Fig. 10e illustrates a perspective view from beneath of the system following the folding of the first and second sections to occlude the arrays of microneedles;
Fig. 11a illustrates a plan view from above of an alternative embodiment of a securement system according to the invention and in a deployed state with a catheter hub retained therein;
Fig. 11b illustrates a side elevation of the securement system of Fig. 11a which shows the securement system in contact with and secured to a section of skin;
Fig. 11c illustrates a sectioned side elevation of the system as shown in Fig. 11a and 11b;
Fig. 12a illustrates a plan view from above of the system shown in Fig. 11 and in a partially released or undeployed state;
Fig. 12b illustrates a side elevation of Fig. 12a showing arrays of microneedles of the system partially retracted from the section of skin;
Fig. 12c illustrated a sectioned side elevation of the system as shown in Fig. 12a and 12b;
Fig. 13a illustrates a side elevation of the system and in a further released or undeployed state;
Fig. 13b illustrates a sectioned elevation of Fig. 13a showing arrays of microneedles of the system fully retracted from the section of skin;
Fig. 14a illustrates a plan view of the system in a fully released or undeployed state;
Fig. 14b illustrates a side elevation showing the system in an undeployed state with the arrays of microneedles fully retracted and the system lifted away from the section of skin;
Fig. 14c illustrated a sectioned side elevation of the system as shown in Fig. 14a and 14b;
Fig. 15a illustrates a side elevation of the system and in the fully released or undeployed state with a closure member and a second section of the system separated from one another;
Fig. 15b illustrates a sectioned side elevation of Fig. 15a;
Fig. 16a illustrates a perspective view from above of a further alternative embodiment of a securement system according to the invention and in an undeployed state with a catheter hub located therein;
Fig. 16b illustrates the securement system in a deployed state;
Figure 16c illustrates the securement system follow use and having been translated back into the undeployed state to permit removal of the catheter hub;
Fig. 17a illustrates a perspective view from above of another embodiment of a securement system according to the invention and in an undeployed state with a catheter located therein;
Fig. 17b illustrates the securement system in a deployed state; and
Figure 17c illustrates the securement system follow use and having been translated back into the undeployed state to permit removal of the catheter.

### Detailed description of the drawings

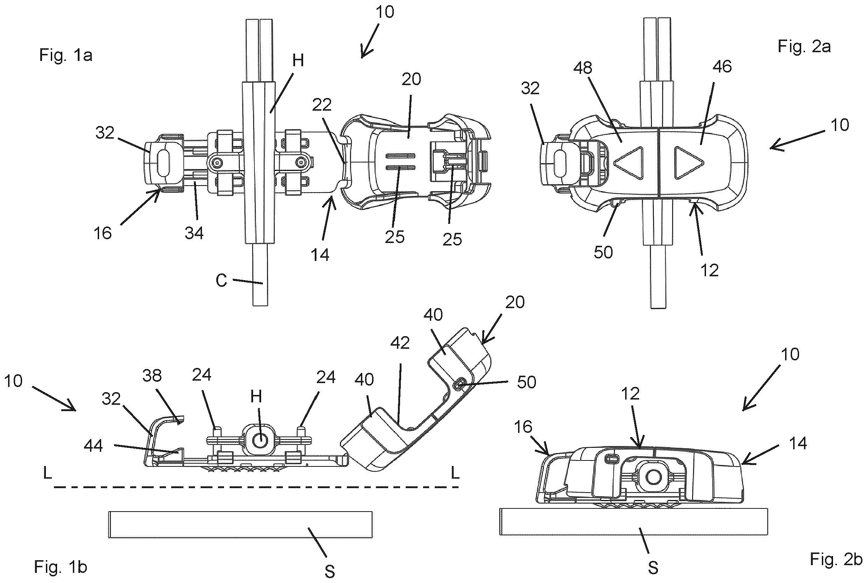

Referring now to Figures 1 to 10 of the accompanying drawings there is illustrated a securement system for medical devices according to a preferred embodiment of the present invention, generally indicated as 10, for use in anchoring a medical device such as a catheter hub H to tissue such as the skin S, dura mater, blood vessels, bowel wall or any other tissue located internally or externally of the body. The securement system 10 may be used with tissue, bone, or any other suitable biological substrate, and may be used in applications such as drug delivery, and/or for securing a biosensor or the like, but has particular application in securing catheter hubs to skin.

The securement system 10 comprises a main body 12 which may be formed of any suitable material, for example a polymer, metal or a composite of materials, and may for example comprise a bioabsorbable material. Although not limited to particular dimensions, in the exemplary embodiment illustrated the body 12 has a length in the region of 20-30mm as measured along a longitudinal axis LL (see Figure 1b), a width in the region of 15-20mm as measured perpendicular to the longitudinal axis LL, and a thickness in the region of 6-10mm perpendicular to both the length and width, and in use measured in a direction perpendicular to the surface of the skin S. These dimensions may of course vary, in particular to suit particular surgical indications. For ease of reference, hereinafter measurements along the length (axis LL) will be referred to as being an "X" coordinate, measurements along the width will be referred to as being a "Y" coordinate and measurements along the depth will be referred to as being a "Z" coordinate.

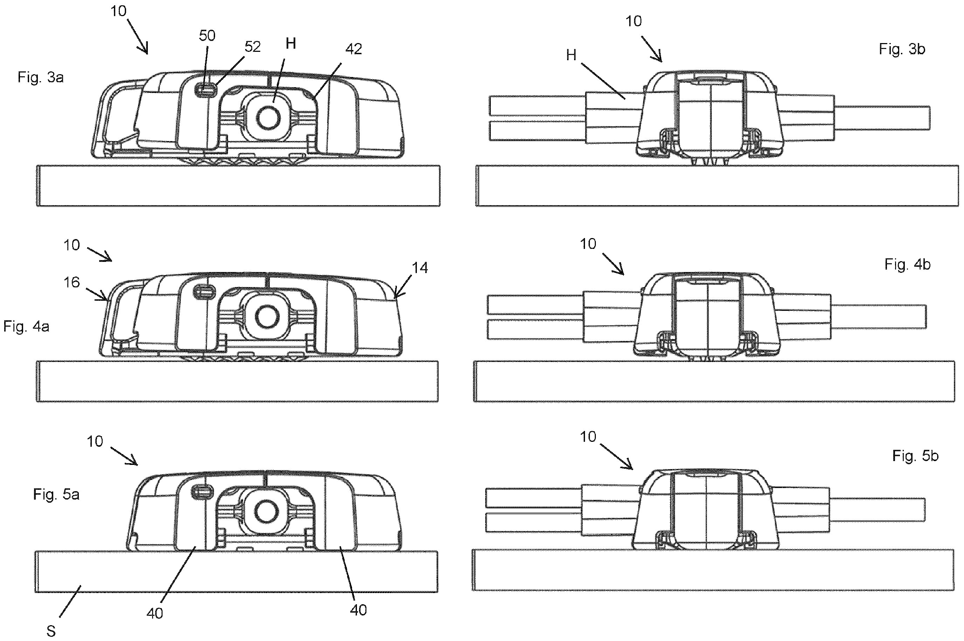

The body 12 comprises a first section 14 and a second section 16 which are displaceable relative to one another in a first "X" direction and between an un-deployed state as illustrated in Figures 1a and 1b and a fully deployed state as illustrated in Figures 5a and 5b, with various intermediate stages in between and as will be described in greater detail hereinafter. The first section 14 and the second section 16 are inter-engagable with one another, and in the embodiment illustrated the first section 14 comprises a first base 18 including one or more channels or guideways (not shown) extending longitudinally therethrough which are shaped and dimensioned to slidingly receive the second section 16 therein, such that the two sections 14, 16 are effectively telescopically displaceable relative to one another. It will of course be appreciated that any other suitable arrangement or configuration may be employed in order to permit relative movement between the first section 14 and the second section 16. While in the embodiment illustrated the first and second sections 14, 16 are reversibly displaceable relative to one another, in other embodiments the displacement may be irreversible.

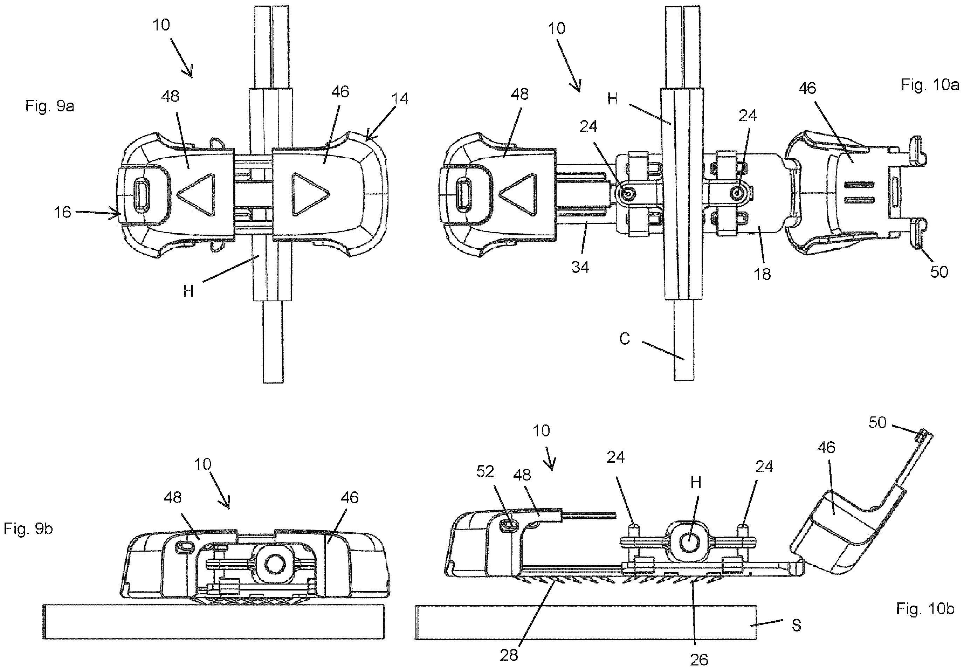

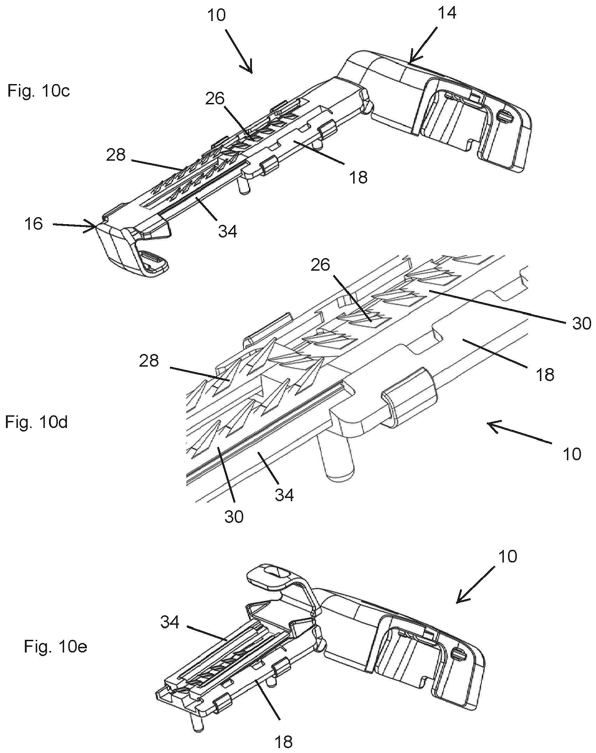

The securement system 10 further comprises a closure member 20 forming part of the first section 14 and hingedly mounted to the first base 18 via a hinge 22 and displaceable between an open position as illustrated in Figures 1a, 1b, 10a and 10b and a closed position as illustrated in Figures 2 to 9. In the open position the closure member 20 permits access to a retention device in the form of a pair of upright and spaced apart posts 24 projecting upwardly in the "Z" direction from the first section 14. The posts 24 are spaced a distance equal to the spacing between a pair of suture holes provided on the catheter hub H, and are of a diameter suitable to receiving the suture holes in order to allow the hub H to be located onto the posts 24 as illustrated such as to initially secure the hub H to the system 10, while the closure member 20 is in the open position exposing the posts 24. In the embodiment illustrated the catheter hub H and associated catheter(s) C extend in a transverse or "Y" direction relative to the main body 12 of the system 10. The posts 24 are preferably mounted such that the distance therebetween may be varied such as to accommodate variations in the spacing between the suture holes on various catheter hubs. The closure member 20 is provided with two sets of longitudinally extending guiderails 25 on the underside of the closure member 20 and defining a longitudinally ("X" direction) extending channel between each set of guiderails 25. Each set of guiderails 25 is located to capture a top of a respective one of the posts 24 in the channel defined between the guiderails 25. The elongate channel between the guiderails 25 permits longitudinal displacement of the closure member 20 during displacement of the system 10 between the undeployed and deployed states while containing the top of the post 24 and thus resisting lateral deflection of the posts 24, to ensure catheter loading translates to shear loading of the posts 24 as opposed to pure bending, thereby increasing the mechanical retention of the catheter hub H and catheter C.

The hinge 22 is preferably provided as a living hinge allowing the first base 18 and closure member 20 to be manufactured as a single component. It is however also envisaged that the closure member 20 could be displaceable between the open and closed positions by any other suitable arrangement or displacement, for example the closure member 20 could be slidable linearly or otherwise between the open and closed positions. Further aspects of the configuration and operation of the closure member 20 will be described in detail hereinafter.

Both the first section 14 and the second section 16 comprise a tissue adhering element in the form of an array of microneedles 26, 28 projecting respectively from the first section 14 and from the second section 16. In each case the microneedles 26, 28 extend from an underside or tissue contacting surface 30 of the respective first section 14 and second section 16. The microneedles may be arranged in a large number of possible configurations. Various configurations of the microneedles 26, 28, along with details of the operation of the microneedles 26, 28 in securing a substrate to tissue are described and shown in earlier patent publications WO2018/069543 and WO 2019/201903 the relevant details of which are incorporated herein by reference. A detailed explanation of the operation of the microneedles 26, 28 is therefore not deemed necessary in the present application.

The microneedles 26, 28 are preferably inclined at an acute angle of inclination relative to the "X" plane in which the tissue contacting surface 30 lies and extend predominantly in the same direction, from root to tip along a major axis of the microneedles 26, 28, as the direction of relative movement between the first section 14 and the second section 16, namely the "first" or "X" direction substantially parallel to the longitudinal axis LL of the body 12. In other words the microneedles 26, 28 can be said to have a greater "X" dimension component than "Z" dimension component.

The microneedles 26, 28 are dimensioned, in the preferred embodiments illustrated, with an axial length from root to tip of approximately 2mm and a depth or "Z" coordinate length of approximately 0.9mm. It has also been found that the preferred angular inclination of the microneedles 26, 28 is between 15° and 50°, more preferably between 20° and 30°, and most preferably approximately 26.5° to the "X" plane. Both the "X" coordinate length the "Z" coordinate length of each microneedle 26, 28 will vary depending on the angular inclination thereon. It will of course be appreciated that all of these dimensions are exemplary and may vary, in particular to suit different surgical or medical applications or tissue types.

The microneedles 26, 28 are arranged and oriented such that the microneedles 26 protruding from the first section 14 extend or point in a direction generally opposite to that of the microneedles 28 protruding from the second section 16. In this way the microneedles 26 essentially face or oppose the microneedles 28. It is also preferable that at least one of the microneedles 26 overlaps with at least one of the microneedles 28 in the "X" and "Z" directions, at least when the securement system 10 is in either the deployed and/or un-deployed state, but most preferably when in the deployed state. The microneedles 26, 28 are preferably spaced from one another in the "X" direction such that the tip of any one microneedle 26, 28 just reaches or may slightly overlap with the root of the adjacent microneedle 26, 28.

The microneedles 26, 28 are arranged to penetrate at least an upper layer or region of the skin S or other tissue to which the system 10 is to be secured, initially by pressing the tissue contacting surface 30 of the body 12 downwardly onto the anchorage site on the skin S in order to push the microneedles 26, 28 onto the tissue in a minimally invasive manner. The securement system 10 is applied to the anchor site in the un-deployed state as illustrated in Figures 1a and 1b in which the first and second sections 14, 16 are drawn outwardly from one another. The catheter hub H is mounted to the posts 24 with the closure member 20 in the open position and the closure member then rotated into a partially closed position as illustrated in Figures 2a and 2b, wherein the system 10 is located onto the deployment site on the skin S. Once the microneedles 26, 28 are engaged against the skin S the first and second sections 14, 16 are manually displaced relative to one another into a deployed state, with this process being illustrated in stages in in Figures 3 to 5. The relative displacement of the first and second sections 14, 16 from the un-deployed to deployed state results in the microneedles 26, 28 being drawn into the skin S as shown in Figures 4 and 5.

By providing the opposed sets of microneedles 26, 28 the local region of tissue on which the securement system 10 is deployed is effectively captured and lightly compressed and stretched between the overlapping microneedles 26, 28 in order to apply shear deformation and thereby robustly secure the securement system 10 in position. In particular when the securement system 10 is displaced into the deployed state the local region of tissue beneath the body 12 is elastically deformed or compressed and stretched by the displacement of the first section 14 relative to the second section 16, and thus by displacement of the microneedles 26 relative to the preferably overlapping microneedles 28. This elastic shear deformation of the tissue results in a reactive force being applied by the tissue against the microneedles 26, 28 thereby actively engaging and retaining the tissue surrounding the microneedles 26, 28. As a result the microneedles 26, 28 do not need to penetrate to a significant depth to achieve the necessary retention, and may for example be of a length in the region of 0.1-5mm from root to tip, and have a depth of penetration of less than 1000µm, although again this dimension may be varied as required. As a result, for skin-based indications, the microneedles 26, 28 can be dimensioned such as not to penetrate to the depth of most pain receptors and blood vessels.

In order to retain the securement device 10 in the deployed state such as to prevent or reduce the possibility of unintended displacement of the catheter hub H and the catheter C during use, the first and second sections 14, 16 are preferably releasably lockable relative to one another when in the deployed state. The second section 16 comprises a receiving section 32 formed integrally with and extending upwardly from a second base 34 which together form the second section 16. The receiving section 32 is curved or otherwise shaped to define a space or cavity for receiving the adjacent end of the closure member 20 of the first section 14 as the first and second sections are displaced into one another as they are translated from the undeployed to the deployed states. The closure member 20 is provided with a first coupling comprising a recess 36 formed integrally with the closure member 20 in an upper face thereof and adjacent the free end which engages with the receiving section 32. The receiving section 32 defines a second cooperating coupling in the form of a barbed latch 38 formed at the free end of the receiving section 32 and engageable in the recess 36. The first coupling further comprises a secondary latch 38a formed in the closure member 20 longitudinally spaced from the recess 36, with second coupling further comprising a complementary secondary recess 36a defined by the receiving section 32 and into which the secondary latch 38a is engaged once the closure member 20 is fully engaged with the receiving section 32.

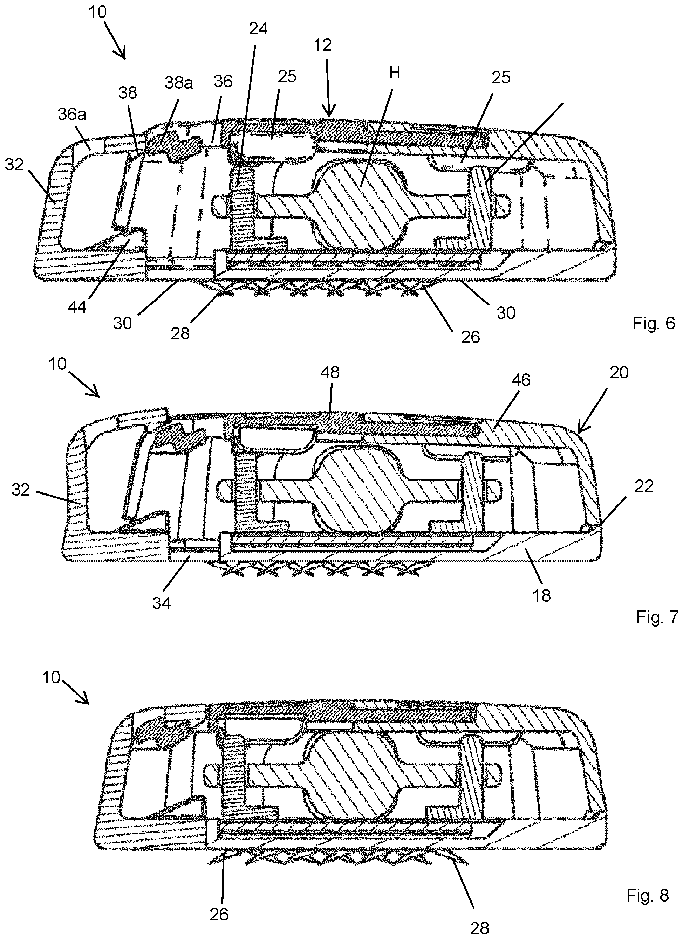

As illustrated in the sequence of Figure 6 to 8 as the first and second sections 14, 16 are translated from the undeployed state (Figure 6) to the deployed state (Figure 8) the free end of the closure member 20 contacts the cantilevered free end of the receiving section 32. The free end of the receiving section 32 is resiliently deformable and thus the barbed latch 38 deforms upwardly in the "Z" direction to slide over the end of the closure member 20 and along the upper surface therefore before reaching the recess 36. The resilience of the free end of the receiving section 32 biases the barbed latch 38 into the recess 36. The same displacement will bring the secondary latch 38a into engagement with the secondary recess 36a. The latches and recesses together serve to lock the first and second sections 14, 16 in the deployed state thus ensuring the microneedles 26, 28 remain suitably engaged in the skin S.

In order to counter the relatively small footprint of the arrays of microneedles 26, 28 the securement device is preferably provided with one or more abutments or stabilising feet 40 which engage the skin S or other surrounding tissue, preferably outboard of the microneedles 26, 28 defining the contact patch on the skin contacting surface 30, in order to provide one or more outriggers which resist any rolling or pivoting of the body 12 about the contact patch, which could otherwise act to disengage one or more of the microneedles 26, 28 or cause relative movement of the catheter C to the skin S at the catheter insertion site (not shown). In the preferred embodiment illustrated the stabilisation system 10 comprises four feet 40, two on either side of the body 12 and preferably formed one on either side of an opening 42 in a sidewall of the closure member 20 through which, in use, the catheter hub H and/or catheter C extend. Each stabilising foot 40 is effectively defined by a lower edge of the sidewall of the closure member 20, although other suitable configurations may of course be employed.

However it is preferable that the stabilising feet 40 only come into contact with the skin S after the arrays of microneedles 26, 28 have been fully deployed or inserted into the skin S. This will ensure that the downward pressure applied by the user is concentrated on the microneedles 26, 28 during deployment which will promote initial insertion into the skin S. The securement device 10 is thus beneficially adapted to effect the automatic displacement of the stabilising feet from a location above or out of the plane of the tissue contacting surfaces 30 to a location in or below the plane of the tissue contacting surfaces 30 as the first and second sections 14, 16 undergo the final phase of their relative displacement into the deployed state. This functionality is achieved through the provision of a set of co-operating engaging surfaces on the first and second sections 14, 16 whose relative motion during displacement of the system 10 from the undeployed to the deployed state effects articulation of the closure member 20 from a partially closed state to a fully closed state in which the stabilising feet are in or below the plane of the tissue contacting surfaces 30. This is most clearly illustrated in Figures 4 and 5. Referring to Figures 6 to 8 the above mentioned co-operating engaging surfaces comprise a ramp 44 provided on a lower portion of the receiving section 32 and a lower edge of an end wall of the closure member 20. As the first and second sections move towards the deployed state the lower edge travels downwardly along the ramp 44 and the biasing action of the upper portion of receiving section 32 applying a force on the upper face of the closure member 20 forces the lower edge of the closure member 20 to remain engaged with the ramp 44 during the translation. This has the effect of causing the closure member 20 to rotate from the partially closed position shown in Figure 6 to a fully closed or further rotated position shown in Figure 8 in which the stabilising feet 40 are displaced downwardly in the "X" direction such as to automatically go from the location above or out of the plane of the tissue contacting surfaces 30 to a location in or below the plane of the tissue contacting surfaces 30 and thus contact the skin S to provide additional stability. It should be understood that alternative co-operating engaging surfaces could be employed to achieve the above described functionality.

In order to release the securement system 10 from the skin S when no longer required, it is necessary to displace the microneedles 26, 28 in an opposite direction to that described above, by translating the first and second sections 14, 16 outwardly of one another into the undeployed state. However the recess 36 and latch 38 are engaged and thus act to resist any such displacement of the first and second sections 14, 16. The system 10 is thus designed to simplify removal by avoiding the requirement to release the recess 36 and latch 38, which are very small components and thus not easily manipulated by a user. The closure member 20 is thus formed of a primary portion 46 which defines the hinge 22 with the first base 18, and a secondary portion 48 which is displaceable relative to the primary portion 46 between a retracted position (Figure 1 to 8) and an extended position (Figures 9). In the embodiment illustrated this displacement is linear and the primary and secondary portion 46, 48 may be provided with easily visible iconography (e.g. arrowheads) to illustrate to the user the direction in which displacement occurs.

The first coupling comprising the recess 36 is provided on the secondary portion which is therefore locked to the receiving section 32 of the second section 16. In use the primary and secondary portions 46, 48 are releasably locked to one another by any suitable means. In the embodiment illustrated this releasable locking is achieved by means of a release mechanism defined by a pair of opposed detent locks 50 resiliently fixed to the primary portion 46 and which are biased into a corresponding pair of sockets 52 provided in a sidewall of the secondary portion 48. While the detent locks 50 are located in the sockets 52 the primary and secondary portions 46, 48 are locked together in the retracted position. A user may therefore grasp the system 10 to release same from the skin S, holding one end via the sidewall of the primary portion 46 between the thumb and forefinger of one hand, the other end via the sidewall of the secondary portion 48 between the thumb and forefinger of the other hand, which will naturally engage and thereby depress the pair of detent locks 50 to release same from the sockets 52. The primary and secondary portions 46, 48 of the closure member 20 may therefore be drawn away from one another as illustrated in Figure 9. As the secondary portion 48 and the second section 14 are locked together this will draw the first and second sections 14, 16 away from one another such as to translate the system 10 into the undeployed state. The opposed sidewalls of the closure member 20 are preferably flared outwardly in plan view towards either end of the body 12 such as to define contact points on which a user's thump and forefinger (or any other digits) will naturally contact when gripping the system 10, and at which points the detent locks 50 are located to ensure they are effectively automatically depressed when a user grips the system 10 for removal, again increasing the intuitive operation of the system 10.

The primary and secondary portions 46, 48 are preferably separable when in the extended position as shown in Figures 10a and 10b, allowing the primary portion 46 to be hinged back into the open position to enable the catheter hub H to be released from the system 10 following removal therefore from the skin S. In order to reduce the risk of injury through contact with the arrays of microneedles 26, 28 following removal, the securement system 10 may be adapted to cover or occlude the microneedles 26, 28. As illustrated in Figures 10c to 10e, the first base 18 and the second base 34 may be displaced into a fully extended position as illustrated in Figure 10c and 10d, in which the ends of the first and second base 18, 34 are adjacent one another, and the bases 18, 34 then folded over against one another such that the tissue contact surfaces 30 are in opposing or face to face engagement. This acts to cover both sets of microneedles and thus prevent possible injury. Any suitable lock (not shown) such as a simple clip or the like may be provided to retain the bases 18, 34 in this configuration. The system 10 may then be safely disposed.

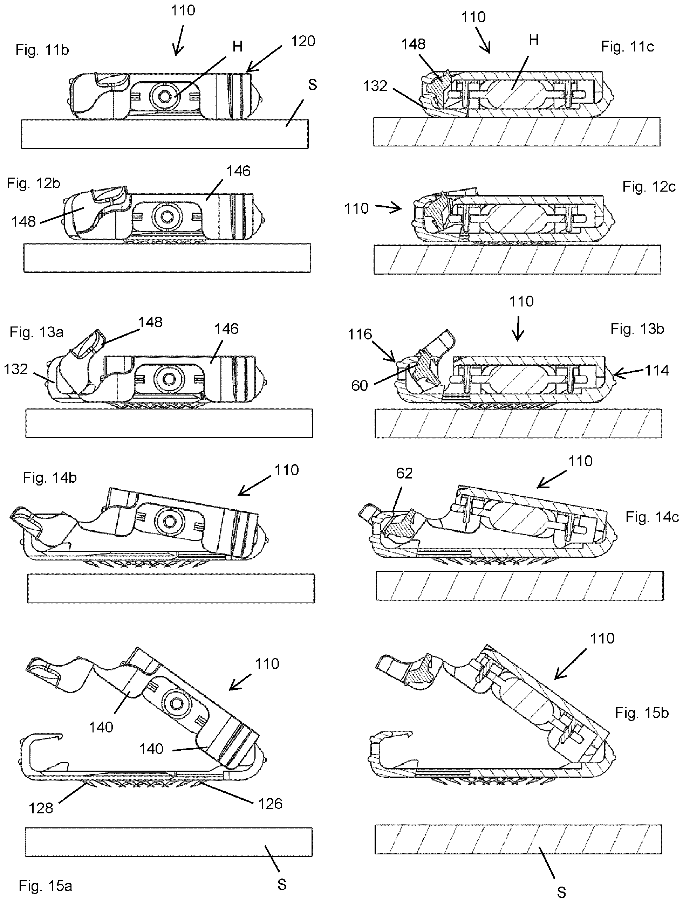

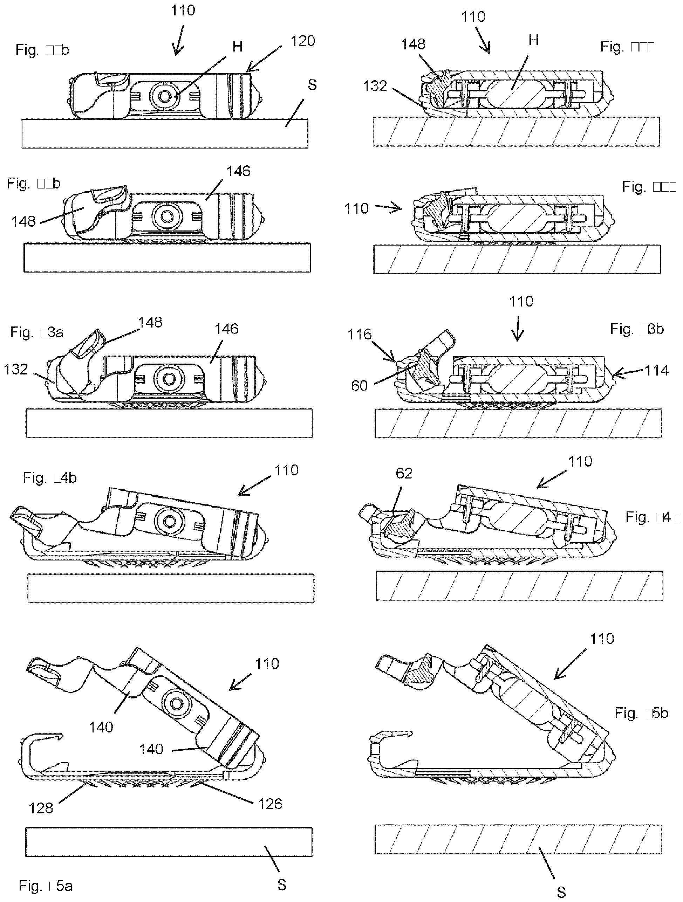

Referring now to Figures 11 to 15 there is illustrated a securement system for medical devices according to an alternative embodiment of the present invention, generally indicated as 110, for use in anchoring a medical device such as a catheter hub H to tissue such as the skin S, dura mater, blood vessels, bowel wall or any other tissue located internally or externally of the body. The securement system 110 may again be used with tissue, bone, or any other suitable biological substrate, and may be used in applications such as drug delivery, and/or for securing a biosensor (not shown) or the like. In this alternative embodiment like components have been accorded like reference numerals and unless otherwise stated perform a like function.

The system 110 comprises a body 112 having a first section 114 and a second section 116 which are translatable between undeployed and deployed states in essentially the identical manner as described above. A closure member 120 allows the catheter hub H to be located in the system 10 and secured in place as described above. Opposed arrays of microneedles 126, 128 are again provide to anchor the system 110 to the skin in exactly the same manner as described in connection with the first embodiment above. The system 110 is again adapted to effect the automatic displacement of stabilising feet 140 from a location above or out of the plane of tissue contacting surfaces to a location in or below the plane of the tissue contacting surfaces as the first and second sections 114, 116 undergo the final phase of their relative displacement into the deployed state. This functionality is achieved in the same manner as with the first embodiment, through the provision of a set of co-operating engaging surfaces on the first and second sections 114, 116 whose relative motion during translation of the system 110 from the undeployed to the deployed state effects articulation of the closure member 120 from a partially closed state to a fully closed state in which the stabilising feet 140 are in or below the plane of the tissue contacting surfaces.

The design of the system 110 differs from the first embodiment in the configuration of the closure member 120 and the operation thereof during release of the system 110 from the skin following use. The closure member 120 includes a primary portion 146 and a secondary portion 148 displaceable relative to the primary portion 146 between a retracted position (Figure 11) and an extended position (Figures 14 and 15) with the intervening sequences in the removal shown in the Figures therebetween. In this embodiment the displacement is rotational with the secondary portion 148 rotatable towards a receiving section 132 of the second section 116. The primary and secondary portions 146, 148 may be provided with easily visible iconography (e.g. arrowheads) to illustrate to the user the direction in which the rotational displacement occurs. As with the first embodiment the first and second sections are locked in the deployed state through engaging and locking the receiving section 132 with the secondary portion 148 of the closure member 120 of the first section 114. In order to facilitate the necessary separation of the first and second sections 114, 116 such as to retract the microneedles 126, 128 from the skin S the secondary portion 148 is grasped by the user and rotated away from the primary portion 146. As more clearly illustrated in the sectioned views of Figure 11c, 12c, 13b, the secondary portion 148 and the receiving section 132 have a set of co-operating engaging surfaces whose relative motion during displacement of the secondary portion 148 from the retracted to the extended positions translates the rotary motion of the secondary portion 148 into linear motion of the second section 116 relative to the first section 114 to translate the system 110 into the undeployed state. The complementary engaging surfaces preferably comprise a cam surface 60 on the secondary portion 148 and a corresponding follower surface 62 on the receiving section 132, although it will be appreciated that other surface profiles may be utilising which provide the above functionality.

As illustrated between Figures 14 and 15, once the first and second sections 114, 116 have been translated into the undeployed state through rotation of the secondary portion 148 into the fully extended position, the locking connection between the receiving section 132 and the secondary portion 148 is released, allowing the closure member 120 to be rotated back into the open position. The catheter hub H can then be extracted once the system 110 has been removed from the skin S.

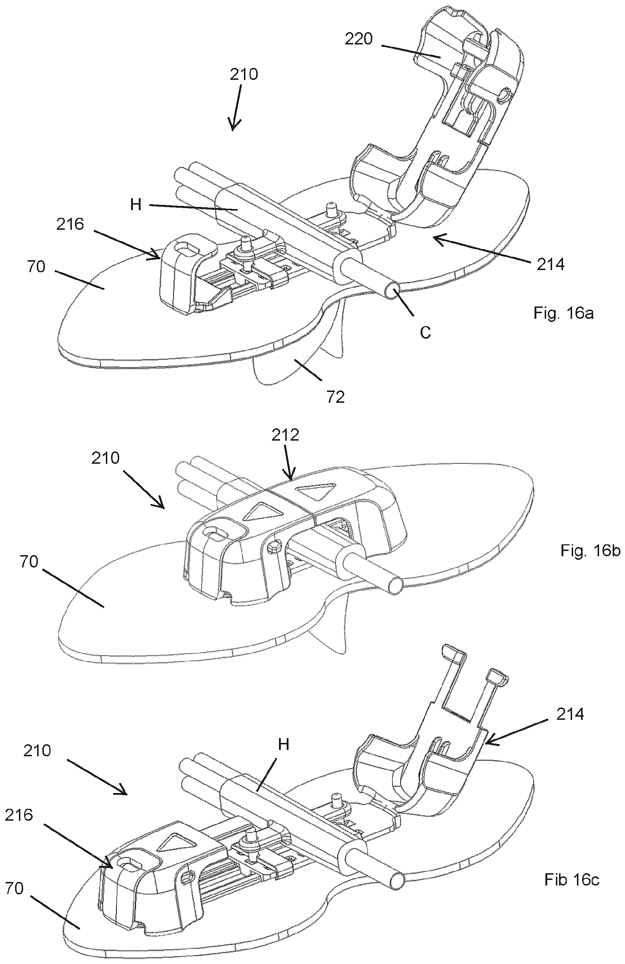

It is also envisaged that the securement device could be provided without the arrays of microneedles, and to use an adhesive layer or patch as the skin adhering element in place of the microneedle arrays, in order to allow a catheter hub to be secured within the system as hereinbefore described, and to then use the adhesive to anchor the system to the skin. Such an embodiment is illustrated in Figure 16 which illustrates a securement system for medical devices according to an further alternative embodiment of the present invention, generally indicated as 210, for use in anchoring a medical device such as a catheter hub H to tissue such as the skin or any other tissue located internally or externally of the body. In this further alternative embodiment like components have been accorded like reference numerals and unless otherwise stated perform a like function.

The system 210 comprises a body 212 having a first section 214 and a second section 216 which are translatable between undeployed and deployed states in essentially the identical manner as described above in relation the system 10 of the first embodiment. A closure member 220 again allows the catheter hub H to be located in the system 210 and secured in place as described above. The system 210 differs in the means by which adherence to the skin is achieved. Thus in place of the previously described arrays of microneedles the system 210 comprises a skin adhering element in the form of an adhesive patch 70 which is fixed to a skin contacting surface of either the first or second section 214, 216, preferably the first section 214 as in the illustrated embodiment. The second section 216 is therefore capable of underdoing the above described displacement relative to the first section 214 as it is not secured to the adhesive patch 70. In the particular embodiment illustrated the adhesive patch 70 has a footprint larger than the system 210 in order to provide sufficient skin adherence. It will be appreciated that the size and shape of the patch 70, in addition to the composition of the adhesive used, may be varied as required. A conventional release liner 72 is preferably provided on the underside or skin contacting side of the adhesive patch 70.

The catheter hub H or other medical device may thus be captured within the system 210 prior to location on the skin. The system 210 is initially in the undeployed state and the closure member 220 is opened as illustrated in Figure 16a. The hub H is located in the system 210 and, referring to Figure 16b, the closure member 220 is displaced into the closed position and the first and second sections 214, 216 are translated into the deployed state as hereinbefore described with reference to the previous embodiments. This acts to lock the first and second sections 214, 216 together, with the hub H captured within the system 210, with stabilising feet 240 having been automatically engaged against the adhesive pad 70 to provide additional stability in the same manner as described for the system 10 of the first embodiment.

At this point the release liner 72 may be removed and the system 210 adhered to the skin at the necessary location, robustly securing the system 210 in place so as to secure the catheter hub H and any associated catheter C. Once the hub H is no longer required, the system 210 may be displaced into the undeployed state as described above in connection with the system 10 of the first embodiment, in order to allow the closure member 20 to be reopened and the hub H to be released. As illustrated in Figure 16c, as the second section 216 is not fixed to the adhesive patch 70 it will extend over the adhesive patch 70 as it is displaced relative to the first section 214. The adhesive patch 70 may then be removed from the skin in the conventional fashion.

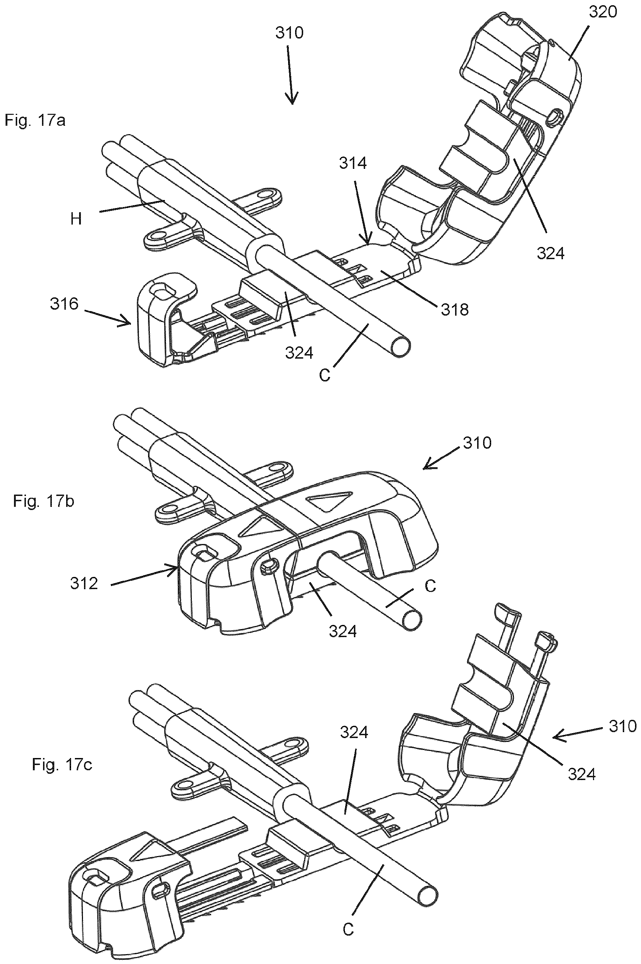

Although the above embodiments have been described and shown as securing a catheter hub H therein, as mentioned the system may be adapted to directly capture and retain a catheter C or similar line therein. Thus referring to Figure 17 there is illustrated a modified securement system for medical devices according to an alternative embodiment of the present invention, generally indicated as 310, for use in anchoring a catheter C (which may or may not have a hub H associated therewith) to tissue such as the skin or any other tissue located internally or externally of the body. In this alternative embodiment like components have been accorded like reference numerals and unless otherwise stated perform a like function.

The securement system 310 is essentially identical in configuration and operation to the system 10 of the first embodiment, including a body 312 having a first section 314 and a second section 316 which are translatable between undeployed and deployed states in essentially the identical manner as described above in relation to the system 10. A closure member 320 allows the catheter C to be located in the system 310 and secured in place as described above. The system 310 differs in the means by which the catheter C is retained, and in place of the posts 24 of the system 10 a retention device in the form of a split clamp 324 is provided with a transversely extending ("Y" direction) channel formed therein for receiving and securely clamping the catheter C. One half of the split clamp 324 is located on the underside of the closure member 320 and the other half is located on a first base 318 of the first section 314. Thus the system 310 is initially provided in the undeployed state as illustrated in Figure 17a and the catheter C is laid in the channel of the half of the split clamp 324 on the first base 318. The closure member 320 is then rotated into the closed position and the system 310 translated into the deployed state as illustrated in Figure 17b in order to enclose and securely capture the catheter C. The system 310 may be translated back into the undeployed state as hereinbefore described, and as illustrated in Figure 17c, in order to release the catheter C therefrom.

The medical device securement system 10; 110; 210; 310 of the present invention thus provides a discrete yet robust and pain free means of securing a medical device such as a catheter hub H to the skin or other tissue of a patient, and whose removal is simple, quick and intuitive thereby providing a significant benefit to health care workers and the like by saving time and effort in both applying and removing the system 10; 110; 210; 310.

## Claims

1. A medical device securement system comprising a main body having a first section and a second section displaceable relative to one another to translate the system between an undeployed state and a deployed state; a tissue adhering element provided on at least one of a tissue contacting surface of the first section and a tissue contacting surface of the second section; a retention device provided on the main body for receiving and engaging the medical device; wherein the first section comprises a first base defining the tissue contacting surface and a closure member hingedly articulated relative to the first base between a closed position defining an enclosure between the first base and the closure member in which the retention device is located and in which the medical device may be at least partially contained, and an open position permitting access to the enclosure to facilitate insertion/removal of the medical device.

2. A medical securement device according to claim 1 in which the tissue adhering element comprises a first array of microneedles projecting from the tissue contacting surface of the first section and a second array of microneedles projecting from the tissue contacting surface of the second section.

3. A medical device securement system according to claim 1 or 2 in which the first and second sections are displaceable relative to one another to translate the system into the deployed state only when the closure member is in the closed position.

4. A medical device securement system according to any of claims 1 to 3 in which the first section comprises a first coupling and the second section comprises a second coupling engageable with the first coupling to lock the system in the deployed state.

5. A medical device securement system according to claim 4 in which the first and second couplings are arranged to be engaged with one another as the system is displaced into the deployed state.

6. A medical device securement system according to claim 4 or 5 in which the first and second couplings are arranged for irreversible interlocking with one another.

7. A medical device securement system according to any preceding claim in which the closure member comprises a primary portion hingedly articulated to the first base and a secondary portion displaceable relative to the primary portion between a retracted position and an extended position, the secondary portion and the second section being engaged when the system is in the deployed state, the secondary portion being displaceable from the retracted position to the extended position while remaining engaged with the second section such as to displace the system into the undeployed state.

8. A medical device securement system according to any preceding claim in which the closure member comprises a release mechanism operable to retain the primary and secondary portions in the retracted position and to permit the primary and secondary portions to be displaced into the extended position upon actuation of the release mechanism.

9. A medical device securement system according to claim 8 in which the release mechanism comprises a detent lock on one of the primary or secondary portion and a corresponding socket on the other of the primary or secondary portion, the detent lock being biased into the socket when the primary and secondary portions are in the retracted position and manually displaceable out of the socket to permit the primary and secondary portions to be displaced into the extended position.

10. A medical device securement system according to any of claims 7 to 9 in which the secondary portion is displaceable linearly between the retracted and extended positions in order to effect linear displacement of the second section relative to the first section to translate the system into the undeployed state.

11. A medical device securement system according to any of claims 7 to 10 in which the primary and secondary portions are separable when in the extended position.

12. A medical device securement system according to any of claims 7 to 9 in which the secondary portion is rotationally displaceable between the retracted and extended positions, the second section and the secondary portion defining a first set of complementary engaging surfaces which translate rotary motion of the secondary portion from the retracted to the extended positions into linear motion of the second section relative to the first section to translate the system into the undeployed state.

13. A medical device securement system according to claim 12 in which the first set of complementary engaging surfaces comprise a cam surface on one of the first or second section and a corresponding follower surface on the other of the first or second section.

14. A medical device securement system according to any preceding claim in which the first section and the second section comprise a second set of co-operating engaging surfaces whose relative motion during displacement of the system from the undeployed to the deployed state effects articulation of the closure member from a partially closed state to a fully closed state, wherein in the partially closed state one or more abutments on the closure member are located above or out of a plane of the tissue contacting surfaces and in the fully closed state are located in or below the plane of the tissue contacting surfaces.

15. A medical device securement system according to claim 14 in which the second set of co-operating engaging surfaces comprise a ramp on one of the first or second section and a corresponding follower on the other of the first or second section, the ramp and follower being positioned such that displacement of the first section relative to the second section displaces the follower along an inclined face of the ramp such as to effect articulation of the closure member from the partially closed state to the fully closed state.

16. A medical device securement system according to claim 14 or 15 in which the closure member comprises a plurality of stabilising feet defining the abutments.

17. A medical device securement system according to claim 16 in which the plurality of stabilising feet are each defined by a lower or free edge of a pair of sidewalls of the closure member.

18. A medical device securement system according to claim 16 or 17 in which the plurality of feet are disposed outboard of the first and second tissue contacting surfaces.

19. A medical device securement system according to any preceding claim in which the second section comprises a second base engaged with and capable of displacement relative to the first base.

20. A medical device securement system according to claim 19 in which the second base defines a channel into which the first base is engaged, the channel being dimensioned to permit the first base to be displaced longitudinally within the channel.

21. A medical device securement system according to any preceding claim in which the retention device comprises a pair of posts in spaced relationship to one another.

22. A medical device securement system according to claim 21 in which the retention device is arranged to enable the relative position of the pair of posts to be adjusted.

23. A medical device securement system according to any preceding claim in which the closure member defines a pair of opposed openings through which the medical device may extend.

24. A medical device securement system according to any of claims 2 to 23 in which the first and second section are displaceable relative to one another in a first direction and one or more microneedles of the first array overlap with one or more microneedles of the second array in a second direction substantially perpendicular to the first direction when the system is in the deployed state.

25. A medical device securement system according to any preceding claim adapted to facilitate occlusion of the tissue adhering element.

26. A medical device securement system according to any preceding claim in which the two tissue contacting surfaces are displaceable into opposing engagement with one another such as to occlude the tissue adhering element.

27. A method of releasably securing a medical device to tissue with a securement system, the method comprising the steps of engaging the medical device with a retention device on a body of the system; displacing a closure member of the securement system from an open position exposing the retention device to a closed position at least partially occluding the retention device and medical device; displacing a first section of the body relative to a second section of the body to translate the securement system from an undeployed state to a deployed state; and securing to the tissue a tissue adhering element provided on at least one of a tissue contacting surface of the first section and a tissue contacting surface of the second section before, during, or after displacing the securement system into the deployed state.

28. A method of releasably securing a medical device to tissue according to claim 27 in which the tissue adhering element comprises a first array of microneedles projecting from the first section and a second array of microneedles projecting from the second section, the method comprising the steps of inserting the first array and the second array of microneedles into the tissue; displacing the first section relative to the second section to translate the securement system from the undeployed state to the deployed state, wherein the first and second array of microneedles effect localised deformation of the tissue surrounding the microneedles when the body is in the deployed state.

29. A method of releasably securing a medical device to tissue according to claim 27 or 26 comprising stabilising the securement system against the tissue by displacing one or more stabilising feet on the first section into contact with the tissue and maintaining said contact while the system is in the deployed state.

30. A method of releasably securing a medical device to tissue according to claim 29 comprising the step of utilising the relative motion of the first and second sections during translation of the system into the deployed state to effect the displacement of the one or more stabilising feet into contact with the tissue.

31. A method of releasably securing a medical device to tissue according to any of claims 27 to 30 comprising subsequently releasing the medical device from the tissue, the method comprising displacing a secondary portion of the closure member relative to a primary portion of the closure member from a retracted position to an extended position, the secondary portion and the second section being engaged when the system is in the deployed state, and displacing the secondary portion from the retracted position to the extended position while the secondary portion remains engaged with the second section such as to displace the system into the undeployed state.

32. A method according to claim 31 comprising the step of displacing the two tissue contacting surfaces into opposing engagement with one another such as to occlude the tissue adhering element.
